# EUROPEAN PATENT SPECIFICATION

(11) **EP 0 347 496 B1**
(45) Date of publication and mention of the grant of the patent: **13.07.1994**
(21) Application number: 88202576.0
(22) Date of filing: 16.11.1988
(51) Int. Cl.: A61L 27/00

(54) **Method for treating material for implantation and implant**
Verfahren zur Behandlung von Implantatmaterial und Implantat
Procédé de traitement d'un matériau d'implantation et implant

(30) Priority: 21.06.1988 EP 88201279
(43) Date of publication of application: 27.12.1989
(73) Proprietor: Boon, Mathilde Elisabeth, NL-9304 TA Lieveren (NL); Kok, Lanbrecht Piet, NL-9304 TA Lieveren (NL)
(72) Inventor: Boon, Mathilde Elisabeth, NL-9304 TA Lieveren (NL); Kok, Lanbrecht Piet, NL-9304 TA Lieveren (NL); Visser, Cornelis Elise, NL-1412 AV Naarden (NL)
(74) Representative: Hoijtink, Reinoud

(56) References cited:
- EP-A- 0 015 055
- WO-A-85/00954
- FR-A- 1 317 584
- US-A- 4 456 589

## Description

Resorption of implants made of biological material is one of the problems in obtaining good results in operations. On the other hand implants of synthetic material bring the danger of infection in the long term. The present invention relates to a method of treating implants of biological material prior to implantation thereof.

Various proposals have been made for the treatment of implants prior to implantation. At the moment allogenous rib cartilage is generally kept immersed in merthiolate® or cialite® at 4°C. Various other methods are described previously for preparing tissue for implantation.

The European patent application 15.055 describes a method for contaminating and/or sterilizing proteinaceous material that might be used as material for implantation. Said method comprises freeing the material from moisture and subjecting the dried material to microwave energy.

The American Patent 4,456,589 describes a method for preparing animal tissue for implant use in humans comprising the step of tanning the tissue chemically or after a preliminary dehydration step by irradiation. The tissue is either irradiated with beta or gamma radiation or chemically tanned with e.g. glutaraldehyde but never both chemically tanned and irradiated. Said Patent does not mention microwave radiation.

WO 85/00954 describes a method for enhancing the acceptance of transplanted organs or tissues by treating the organs or tissue with a suitable dose of ultraviolet radiation.

The present invention has for its object to provide an improved method for treating biological material for implantation prior to implanting.

The present invention therefore provides a method for treating biological material by subjecting the biological material to microwave radiation, characterized in that the biological material is subjected to microwave radiation during a predetermined period of time while being immersed in a liquid reagent, selected from a group comprising alcohol, glutaraldehyde, glycerin.

Initial tests have been carried out on rib cartilage to be implanted into a rabbit. A first area of application appears to be the replacement of nasal septums.

Applications are currently being researched in which skin is treated according to the present invention for repairing physical damage after burn injuries for example.

Other known techniques such as decalcification with HCl are directed at the denaturing of proteins or cross-linking of collagenous molecules as a result of which a probable decrease of the antigenic properties of the implant is achieved.

Internally generated heat can be used through microwave for the denaturing of proteins and at the high temperatures thus achieved the diffusion velocity of reagents is increased in addition to the chemical reaction speed. Obtained through a homogeneous rise in temperature is a denaturing of protein and the formation of disulphide bridges in aggregated protein. As a result tissue that has been exposed to microwave radiation probably contains a coarse network of coagulated protein through which fixatives diffuse more easily. The extent of polymerization of glutaraldehyde can also be improved with microwave radiation. The toxic effect on the surrounding tissue is hereby avoided. Also, the cross-linking becomes more complete.

A sterile implant can be manufactured in this way and in particular the retro-viruses (AIDS virus) can be eliminated. The temperature of 60°C reached during the treatment and the presence of alcohol also reduce the virulence of the retro-virus (AIDS virus) such that infection of the recipient with virus is substantially excluded.

The experiments described hereinafter were carried out in a standard kitchen microwave oven; other experiments were performed in a Biorad® H2500 microwave oven which is now commercially available and which enables a good temperature control in the interior of the microwave oven.

The present invention wil be elucidated with reference to practical experiments performed under the experimental conditions given in the following table.

**TABLE**

| DIFFERING MICROWAVE TREATMENTS | | | | |
|---|---|---|---|---|
| Experiment number | solution | radiation duration | temp. | dose (Watt) |
| 1 | Cialite® | 5 minutes | 50°C | 150 |
| 2 | Cialite® | 20 minutes | 50°C | 150 |
| 3 | Cialite® | 10 minutes | 80°C | 450 |
| 4 | alcohol | 5 minutes | 60°C | 150 |
| 5 | alcohol | 10 minutes | 60°C | 150 |
| 6 | dry | 3 minutes | 60°C | 150 |
| 7 | (not radiated) | | | |

Rib cartilage implants taken from fresh human skeletons were treated or refined under the conditions mentioned in the above table. The rib cartilage was cleaned of adhering tissue, whereby the perichondrium remained intact. Microwave radiation was applied to the 3 mm thick pieces of rib cartilage in a Miele M696 in which the immersion liquid, radiation time, temperature and power level applied were varied.

The implants were introduced under anaesthetic into eight adult rabbits of 1.5-1.9 kg in weight. Incisions were made vertically on the spinal column and the various implants were inserted into the subcutaneous tissue of the back. The skin was sutured with vicriel 4x0. No post-operative antibiotic treatment was given. Cartilage implants were removed after 12 and 40 weeks and observed. The results of experiments 1-5 were better than with untreated cartilage (experiment 7).

The radiated cartilage has preserved its normal structure, strength and elasticity and no difference could be observed macroscopically in a comparison with the non-treated cartilage.

The cartilage implants were found during removal to have displaced in the case of various animals, but could be identified easily. After 12 weeks of implantation the implant numbered 7 (not radiated) could easily be distinguished from the other implants. The cartilage displayed central necrosis with nuclear debris while granulocytes were present. Macrophages were concentrated around the edges of the implant and an extensive infiltration of macrophages in the cartilage tissue was clearly observable. In the case of the implants treated with microwave (experiments 1-6) better results could be observed microscopically. It can be stated generally after 12 weeks of implant that the results ran from good to poor as follows: No. 5, No. 4, No. 1, No. 2, No. 3, with No. 6 as the worst.

40 weeks after implantation a greater difference in microscopic appearance could be seen. The non-treated implants once again displayed the most pronounced resorption by macrophages and the cartilage was centrally fragmented with areas of necrotic tissue around the fragments. These changes were less noticeable than 12 weeks after implantation. In the case of the implants exposed to microwave treatment some macrophages were seen around the edges and in some places even in the cartilage, but fragmentation or central necrosis was not observed. There had been hardly any progression in the resorption process as in the untreated implants. The results could be summarised in sequence as follows: No. 4, No. 5, No. 2, No. 1, No. 3, No. 6.

## Claims

1. Method for treating biological material, such as cartilage, bone, skin, tendons, cardiac valves or vascular tissue, prior to implantation thereof by subjecting the biological material to microwave radiation, **characterized in that** the biological material is subjected to microwave radiation during a predetermined period of time while being immersed in a liquid reagent, selected from a group comprising alcohol, glutaraldehyde, glycerin.

2. Method according to any one of the preceding claims, wherein the predetermined period of time lies between 3 and 20 minutes at a power level of 80 to 450 watts.

3. Tissue for implantation obtainable by a method according to any one of the preceding claims.

## Patentansprüche

1. Verfahren zum Behandeln von biologischem Material, wie Knorpel, Knochen, Haut, Sehnen, Herzklappen oder Gefäßgewebe, vor seiner Implantation, bei dem das biologische Material einer Mikrowellenstrahlung ausgesetzt wird, dadurch gekennzeichnet, daß das biologische Material der Mikrowellenstrahlung während einer vorgegebenen Zeitdauer ausgesetzt wird, während es in ein flüssiges Reagenz aus der Gruppe Alkohol, Glutaraldehyd und Glycerin, eingetaucht ist.

2. Verfahren nach einem der vorangehenden Ansprüche, bei dem die vorgegebene Zeitdauer zwischen 3 und 20 Minuten bei einem Leistungspegel von 80 bis 450 Watt liegt.

3. Gewebe für Implantationen, das durch ein Verfahren nach einem der vorangehenden Ansprüche erhältlich ist.

## Revendications

1. Procédé pour le traitement de matière biologique, comme du cartilage, de l'os, de la peau, des tendons, des valvules cardiaques ou des tissus vasculaires, avant l'implantation de ceux-ci, en soumettant la matière biologique à un rayonnement micro-onde, caractérisé en ce que la matière biologique est soumise à un rayonnement micro-onde durant une période de temps prédéterminée tandis qu'elle est immergée dans un agent chimique liquide sélectionné dans un groupe comprenant l'alcool, l'aldéhyde glutarique et la glycérine.

2. Procédé selon la revendication 1, dans lequel la période de temps prédéterminée s'étend entre trois et vingt minutes à un niveau de puissance compris entre 80 et 450 watts.

3. Tissu pour implantation que l'on peut obtenir par un procédé selon l'une quelconque des revendications précédentes.
